(19) Europäisches Patentamt / European Patent Office / Office européen des brevets

(11) **EP 2 372 912 A1**

(12) **DEMANDE DE BREVET EUROPEEN**

(43) Date de publication:
**05.10.2011 Bulletin 2011/40**

(51) Int Cl.:
*H03H 21/00* *(2006.01)*    *A61B 5/06* *(2006.01)*

(21) Numéro de dépôt: **11160085.4**

(22) Date de dépôt: **28.03.2011**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**

(30) Priorité: **29.03.2010 FR 1052261**

(71) Demandeur: **Commissariat à l'Énergie Atomique et aux Énergies Alternatives**
**75015 Paris (FR)**

(72) Inventeur: **Lesecq, Suzanne**
**38190, FROGES (FR)**

(74) Mandataire: **Colombo, Michel et al**
**Brevinnov**
**324, rue Garibaldi**
**69007 Lyon (FR)**

(54) **Procede et systeme de calibration, support d'enregistrement pour ce procede**

(57)    Ce procédé de calibration des coefficients d'un observateur d'une variable d'état $x_k$ comporte :
- la mesure (50) d'une variable $z_k$ d'un système physique à N instants différents, cette variable $z_k$ étant fonction de la variable d'état $x_k$,
- la détermination (54) d'un vecteur p de coefficients qui minimise le critère suivant en respectant un ensemble prédéterminé $\Delta$ de contraintes :

$$\left\{ f(p) = \sum_{k=1}^{N} \| z_k - \phi(\hat{x}_k, p) \|^2 \right\}$$

OÙ :
• $\hat{x}_k$ est l'estimation de la variable $x_k$ construite par l'observateur calibré avec les coefficients du vecteur p,
• $\phi$ est une fonction connue qui relie l'estimation $\hat{x}_k$ à une estimation $\hat{z}_k$ de la variable $z_k$,
• $\|...\|$ est une norme, et
• la ou les contraintes de l'ensemble $\Delta$ imposent que la trajectoire de la variable $z_k$ soit comprise dans un couloir d'incertitude situé de part et d'autre de la trajectoire de l'estimation $\hat{z}_k$ au moins pour la majorité des instants $k$.

Fig. 2

EP 2 372 912 A1

**Description**

L'invention concerne un procédé et un système de calibration des coefficients d'un observateur d'une variable d'état $x_k$ d'un système physique. L'invention concerne également un support d'enregistrement pour la mise en oeuvre de ce procédé.

Un observateur permet d'estimer la variable d'état $x_k$ à partir de mesures $y_k$ de grandeurs physiques de ce système, où l'indice k identifie l'instant de mesure.

Il est connu de calibrer les coefficients d'un observateur par expérimentations successives de différents jeux de coefficients jusqu'à ce qu'un jeu de coefficients permette d'obtenir le fonctionnement souhaité de cet observateur. Typiquement, le comportement souhaité de l'observateur est :

- une convergence rapide vers une estimation $\hat{x}_k$ la plus exacte possible de la variable $x_k$, et
- une stabilité élevée.

Cette calibration par expérimentation relève d'une démarche heuristique. Elle est souvent longue et il est difficile d'être sûr que la meilleur calibration possible de l'observateur a été obtenue.

Pour résoudre ce problème, d'autres procédés alternatifs ont été proposés. Par exemple, la demande de brevet CA 2 226 282 décrit un procédé de calibration des coefficients d'un filtre de Kalman à l'aide d'un réseau de neurones. Toutefois, ces procédés alternatifs sont souvent aussi complexes à mettre en oeuvre que le procédé par expérimentation.

L'invention vise donc à proposer un procédé de calibration plus simple à mettre en oeuvre.

Ainsi, elle a pour objet un procédé de calibration des coefficients d'un observateur comportant :

- la mesure d'une variable $z_k$ du système physique à N instants différents, cette variable $z_k$ étant fonction de la variable d'état $x_k$,
- la détermination d'un vecteur p de coefficients qui minimise le critère suivant en respectant un ensemble prédéterminé $\Delta$ de contraintes :

$$\left\{ f(p) = \sum_{k=1}^{N} \| z_k - \phi(\hat{x}_k, p) \|^2 \right\}$$

où :

- $\hat{x}_k$ est l'estimation de la variable $x_k$ construite par l'observateur calibré avec les coefficients du vecteur p à l'instant k,
- $\phi$ est une fonction connue qui relie l'estimation $\hat{x}_k$ à une estimation $\hat{z}_k$ de la variable $z_k$,
- $\|...\|^2$ est une norme de la différence entre $z_k$ et $\phi(\hat{x}_k, p)$, et
- la ou les contraintes de l'ensemble $\Delta$ imposent que la trajectoire de la variable $z_k$ soit comprise dans un couloir d'incertitude situé de part et d'autre de la trajectoire de l'estimation $z_k$ au moins pour la majorité des instants *k*.

Le procédé ci-dessus permet de déterminer plus simplement et sans recours à différentes expérimentations les coefficients permettant un bon fonctionnement de l'observateur. De plus, les coefficients ainsi déterminés permettent de garantir que le critère *f(p)* a été minimisé.

Enfin, l'utilisation de l'ensemble $\Delta$ permet de tenir compte du fait qu'il existe une incertitude sur l'estimation $\hat{z}_k$. Le fait de tenir compte de cette incertitude lors de la détermination du vecteur p qui minimise le critère *f (p)* permet d'obtenir une meilleure calibration que si cette incertitude n'était pas prise en compte. En particulier, des solutions aberrantes qui pourraient minimiser le critère *f (p)* sont éliminées.

Les modes de réalisation de ce procédé de calibration peuvent comporter une ou plusieurs des caractéristiques suivantes :

- ■ l'ensemble $\Delta$ comprend une contrainte qui impose que la variable $z_k$ soit comprise entre $\hat{z}_k$-*a* et $\hat{z}_k$+*b*, où a et b sont des constantes positives prédéterminées;
- ■ les constantes a et b sont proportionnelles à un écart-type $\sigma_{\hat{z}_k}$ représentatif de l'incertitude sur l'estimation $\hat{z}_k$ ;
- ■ l'ensemble $\Delta$ de contraintes comprend une contrainte qui impose que la moyenne des écarts entre la variable $z_k$ et son estimation $\hat{z}_k$ soit inférieure à un seuil prédéterminé $S_1$;
- ■ le seuil $S_1$ est fonction de l'incertitude sur l'estimation de $\hat{z}_k$ ;

■ l'observateur est un filtre de Kalman, un filtre de Kalman étendu ou un filtre de Kalman non parfumé ( ou « Unscented Kalman Filter » en anglais) et les coefficients à calibrer sont les coefficients des matrices de covariances $R$ et Q de ce filtre;

■ les matrices $R$ et $Q$ sont choisies diagonales.

Ces modes de réalisation du procédé de calibration présentent en outre les avantages suivants :

-   imposer que la variable $z_k$ soit comprise entre $\hat{z}_k$-$a$ et $\hat{z}_k$+$b$ réduit l'espace de recherche dans lequel le vecteur p doit être recherché ce qui accélère l'exécution de ce procédé ;
-   utiliser des constantes a et b proportionnelles à l'écart-type $\sigma_{\hat{z}_k}$ améliore la cal ibration,
-   utiliser une contrainte qui impose que la moyenne des écarts en valeur absolue entre la variable $z_k$ et son estimation $\hat{z}_k$ soit inférieure au seuil $S_1$ relâche les contraintes imposées ce qui facilite la détermination d'un vecteur p ;
-   utiliser un seuil $S_1$ qui est fonction de l'incertitude sur l'estimation de $\hat{z}_k$ améliore la calibration ;
-   choisir des matrices $R$ et $Q$ diagonales décroît le nombre de coefficients à déterminer ce qui facilite la calibration.

L'invention a également pour objet un support d'enregistrement d'informations comportant des instructions pour la mise en oeuvre du procédé ci-dessus lorsque ces instructions sont exécutées par un calculateur électronique programmable.

L'invention a également pour objet un système de calibration des coefficients d'un observateur d'une variable d'état $x_k$ d'un système physique à partir de mesures de grandeurs physiques de ce système, où l'indice k identifie l'instant de mesure, ce système comportant :

-   au moins un capteur apte à mesurer une variable $z_k$ du système physique à $N$ instants différents, cette variable $z_k$ étant fonction de la variable d'état $z_{k'}$ et
-   un calculateur apte à déterminer un vecteur p de coefficients qui minimise le critère suivant en respectant un ensemble prédéterminé $\Delta$ de contraintes :

$$\left\{ f(p) = \sum_{k=1}^{N} \| z_k - \phi(\hat{x}_k, p) \|^2 \right\}$$

où :

•   $\hat{x}_k$ est l'estimation de la variable $z_k$ construite par l'observateur calibré avec les coefficients du vecteur p à l'instant k,
•   $\phi$ est une fonction connue qui relie l'estimation $\hat{x}_k$ à une estimation $\hat{z}_k$ de la variable $z_{k'}$
•   $\|...\|^2$ est une norme de la différence entre $z_k$ et $\phi(\hat{x}_k, p)$, et
•   la ou les contraintes de l'ensemble $\Delta$ impose que la trajectoire de la variable $z_k$ soit comprise dans un couloir d'incertitude situé de part et d'autre de la trajectoire de l'estimation $\hat{z}_k$ au moins pour la majorité des instants $k$.

L'invention sera mieux comprise à la lecture de la description qui va suivre, donnée uniquement à titre d'exemple non limitatif et faite en se référant aux dessins sur lesquels :

-   la figure 1 est une illustration schématique de l'architecture d'un système de localisation incluant un observateur et d'un système de calibration de cet observateur,
-   la figure 2 est un organigramme d'un procédé de calibration de l'observateur du système de la figure 1, et
-   la figure 3 est un graphe illustrant la trajectoire d'une variable $z_k$ et de son estimation $\hat{z}_k$.

Dans la suite de cette description, les caractéristiques et fonctions bien connues de l'homme du métier ne seront pas décrites en détail.

Ici, une trajectoire désigne les variations au cours du temps d'une variable. La variable qui varie au cours du temps peut être quelconque. En particulier, cette variable n'est pas nécessairement une position dans l'espace. Par exemple, il peut s'agir d'une température ou de toute autre grandeur physique qui varie au cours du temps.

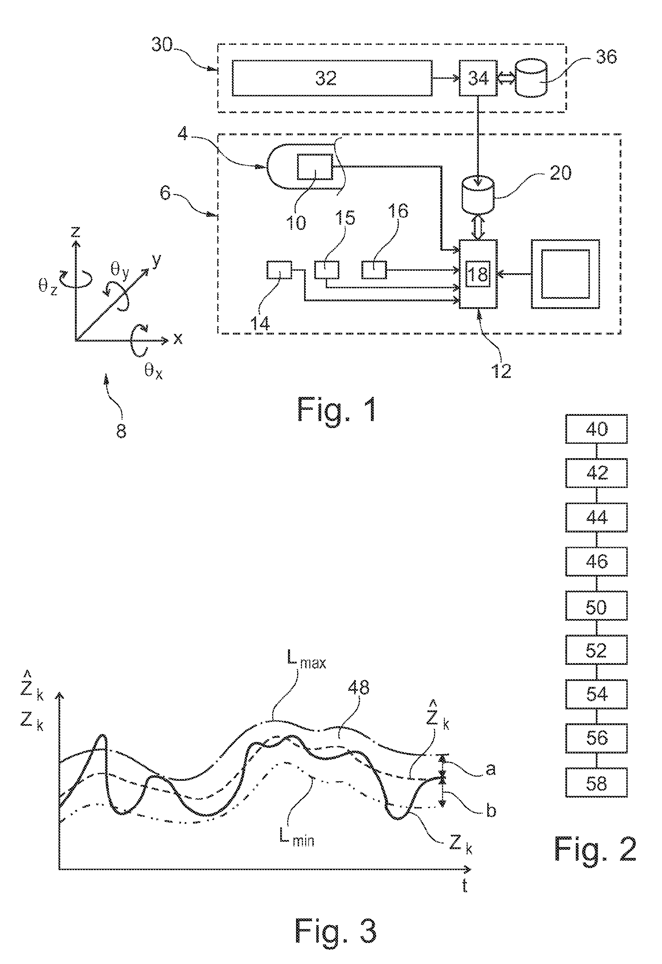

La figure 1 représente un système physique, c'est-à-dire ici un objet 4, et un système 6 de localisation de cet objet 4 dans un référentiel 8.

Par exemple, l'objet 4 est une sonde ou un cathéter introduit dans un corps humain. L'objet 4 est mobile dans le référentiel 8.

Le référentiel 8 est un référentiel fixe présentant trois axes orthonormés X, Y et Z.

La localisation de l'objet 4 dans le référentiel 8 consiste à trouver sa position $x_p$, $y_p$, $z_p$ et son orientation $\theta_x$, $\theta_y$ et $\theta_z$. Les angles $\theta_x$, $\theta_y$ et $\theta_z$ représentent l'orientation de l'objet 4, respectivement, par rapport aux axes X, Y et Z.

Pour localiser l'objet 4, le système 6 comprend ici des sources de champs magnétiques et des capteurs de champ magnétique dont certains sont fixes dans le référentiel 8 et d'autres sont fixés sans aucun degré de liberté à l'objet 4 à localiser. Ici, un capteur 10 de champ magnétique est fixé sans aucun degré de liberté à l'objet 4. Le capteur 10 est par exemple un capteur triaxe, c'est-à-dire un capteur apte à mesurer la projection du champ magnétique sur trois axes de mesure non colinéaires. Un tel capteur mesure la direction du champ magnétique. De plus, généralement, ce capteur mesure également l'amplitude du champ magnétique.

Par exemple, les axes de mesures sont orthogonaux entre eux. Ces axes sont solidaires de l'objet 4.

Ce capteur 10 est raccordé par l'intermédiaire d'une liaison filaire souple à une unité 12 de traitement.

L'unité 12 est également raccordée à trois sources 14 à 16 de champ magnétique. Ces sources sont par exemple des sources triaxes de champ magnétique. Une source triaxe de champ magnétique émet un champ magnétique le long de trois axes d'émission non colinéaires entre eux. Par exemple, une telle source est formée de plusieurs émetteurs monoaxes de champ magnétique alignés, respectivement, sur chacun des axes d'émission de la source. L'émetteur monoaxe émet préférentiellement le champ magnétique le long d'un seul axe. Par exemple, il s'agit d'une bobine dont les spires sont enroulées autour d'un même axe d'émission. Dans ce cas, l'axe d'émission est confondu avec l'axe d'enroulement des spires.

Ici, les sources 14 à 16 sont identiques les unes aux autres. Ces sources sont fixes dans le référentiel 8.

L'unité 12 de traitement alimente les sources 14 à 16 en courant alternatif pour générer le champ magnétique mesuré par le capteur 10. L'unité 12 acquiert également les mesures du champ magnétique réalisées par le capteur 10.

L'unité 12 est équipée d'un observateur 18 qui localise l'objet 4 dans le référentiel 8 à partir des mesures acquises. Ici, l'observateur 18 détermine la position et l'orientation de l'objet 4 en résolvant un système d'équations. Ce système d'équations est obtenu en modélisant les interactions magnétiques entre les sources 14 à 16 et le capteur 10. Dans ce système d'équations, la position $x_p$, $y_p$ et $z_p$ et l'orientation $\theta_x$, $\theta_y$ et $\theta_z$ de l'objet 4 sont les inconnues alors que les valeurs des autres paramètres sont obtenues à partir des mesures réalisées par le capteur 10. Plus d'informations sur de tels systèmes d'équations peuvent par exemple être trouvées dans la demande de brevet EP 1 502 544.

Ici, l'observateur 18 est un filtre de Kalman.

Cet observateur 18 construit une estimation $\hat{x}_k$ d'une variable d'état $x_k$ à partir d'une mesure $y_k$. La variable $x_k$ est un vecteur contenant la position $x_p$, $y_p$, $z_p$ de l'objet 4 et son orientation $\theta_x$, $\theta_y$ et $\theta_z$ dans le référentiel orthonormé 8. La mesure $y_k$ est un vecteur de mesures comprenant les mesures réalisées selon les trois axes de mesure du capteur 10 à un instant $k$.

Les équations du filtre de Kalman de l'observateur 18 sont les suivantes :

$$(1) \qquad \left\{ \begin{array}{c} \hat{x}_{k+1/k} = F_k\, \hat{x}_k \\ P_{k+1/k} = F_k\, P_{k/k}\, F_k^T + Q_k \end{array} \right\}$$

$$(2) \qquad \left\{ \begin{array}{c} \hat{x}_{k+1/k+1} = \hat{x}_{k+1/k} + K_{k+1}\left( y_{k+1} - C_{k+1}\, \hat{x}_{k+1/k} \right) \\ P_{k+1/k+1} = P_{k+1/k} - K_{k+1}\, C_{k+1}\, P_{k+1/k} \end{array} \right\}$$

où :

- $\hat{x}_{k+1/k}$ est l'estimation de la variable $x_k$ obtenue uniquement à partir des données disponibles à l'instant $k$,
- $F_k$ est la matrice qui relie l'estimation $\hat{x}_k$ à l'estimation $\hat{x}_{k+1/k}$,
- $P_{k/k}$ est la matrice d'estimation de la covariance des erreurs sur l'estimation $\hat{x}_k$,
- $Q_k$ est une matrice de covariance du bruit de l'équation du modèle qui contient entre autres les erreurs de modélisation,
- $\hat{x}_{k+1/k+1}$ est l'estimation $\hat{x}_{k+1}$ corrigée à l'instant $k$+1 à partir des mesures réalisées à l'instant $k$+1 ,
- $y_k$ est le vecteur des mesures réalisées à l'instant $k$,
- $C_k$ est la matrice qui relie la variable $x_k$ à la mesure $y_k$,

- $P_{k+1/k+1}$ est la matrice de covariance de l'erreur de l'estimation $\hat{x}_k$ corrigée à partir des mesures $y_k$,
- $K_{k+1}$ est le gain du filtre de Kalman.

La matrice $F_k$ est par exemple obtenue en modélisant les interactions magnétiques entre les sources 14 à 16 et le capteur 10 à l'aide des lois de l'électromagnétisme.

Le gain $K_{k+1}$ du filtre de Kalman est donné par la relation suivante :

$$(3) \qquad K_{k+1} = P_{k+1/k} C_{k+1}^T [ C_{k+1} P_{k+1/k} C_{k+1}^T + R_{k+1} ]^{-1}$$

où $R_{k+1}$ est la matrice de covariance du bruit sur la mesure $y_k$.

Les équations du filtre de Kalman données sont classiques et ne sont donc pas décrites ici plus en détail.

L'utilisation d'un filtre de Kalman permet d'obtenir une estimation précise de la position et de l'orientation de l'objet 4. Toutefois, avant cela, les matrices $R_k$ et $Q_k$ doivent être calibrées c'est-à-dire que les valeurs de leurs coefficients doivent être déterminées. Pour simplifier, on suppose ici que les valeurs de ces coefficients sont constantes. Les matrices $R_k$ et $Q_k$ sont donc également notées $R$ et $Q$ par la suite.

Ici, on note $p_R$ et $p_Q$ les jeux de coefficients, respectivement, des matrices $R$ et $Q$ à déterminer.

Ces jeux $p_R$ et $p_Q$ , une fois le filtre de Kalman calibré, sont enregistrés dans une mémoire 20 raccordée à l'unité 12 de manière à pouvoir être utilisés par l'observateur 18 pour estimer la position de l'objet 4.

La figure 1 représente également un système 30 de calibration de l'observateur 18. Ce système détermine automatiquement les jeux $p_R$ et $p_Q$ de coefficients des matrices $R$ et $Q$ de l'observateur 18.

A cet effet, le système 30 est équipé d'un ou plusieurs capteurs 32 qui mesurent une variable $z_k$. Cette variable $z_k$ est une variable dont la valeur est fonction de la variable $z_k$. Plus précisément, la variable $z_k$ est reliée à la variable $x_k$ par une fonction $\phi$ connue. Cette fonction $\phi$ peut être une fonction linéaire ou au contraire une fonction non linéaire. Par exemple, le capteur 32 mesure l'accélération suivant les axes X, Y et Z de l'objet 4. Dans ce cas, le capteur 32 est formé de plusieurs accéléromètres.

Le capteur 32 peut, dans un autre mode de réalisation, mesurer directement la position de l'objet 4 avec d'autres capteurs que ceux utilisés pour réaliser la mesure $y_k$. Par exemple, le capteur 32 peut comporter à cet effet plusieurs caméras ou d'autres capteurs et d'autres sources de champ magnétique pour mesurer la position de l'objet 4. Dans ce cas particulier, la fonction $\phi$ est la fonction identité.

Le système 30 comporte également un calculateur 34 capable d'acquérir les mesures du capteur 32. Ce calculateur 34 est également apte à calibrer automatiquement l'observateur 18 en déterminant les jeux de coefficients $p_R$ et $p_Q$ à partir des mesures réalisées par le capteur 32.

Dans le cas particulier représenté sur la figure 1, le calculateur 34 est raccordé à la mémoire 20 de manière à pouvoir enregistrer directement dans celle-ci les jeux de coefficients $p_R$ et $p_Q$.

Le calculateur 34 est réalisé à partir d'au moins un calculateur électronique programmable apte à exécuter des instructions enregistrées sur un support d'enregistrement d'informations. Ici, le calculateur 34 est raccordé à une mémoire 36 et cette mémoire 36 contient les instructions nécessaires pour l'exécution du procédé de la figure 2.

Le fonctionnement du système 30 de calibration va maintenant être décrit plus en détail en regard du procédé de la figure 2 et du graphe de la figure 3.

Initialement, lors d'une étape 40, la relation $\phi$ qui relie la variable $z_k$ à la variable $x_k$ est établie. Par exemple, à cet effet, les lois de la physique sont utilisées.

Ensuite, lors d'une étape 42, le nombre de coefficients à utiliser pour calibrer l'observateur 18 est déterminé. Pour cela, des connaissances sur le fonctionnement physique du système 6 sont utilisées. De plus, on applique lors de cette étape le principe de parcimonie qui consiste à limiter autant que possible le nombre de coefficients à calibrer.

Par exemple, dans le cas particulier décrit ici, on admet que les mesures réalisées sur chacun des axes de mesure sont indépendantes les unes des autres. Dans ces conditions, la matrice $R$ est prise diagonale. De même, on suppose que les erreurs sur l'équation d'état sont indépendantes. Dans ces conditions, la matrice Q est prise diagonale. Elles ont donc la forme suivante :

$$(7) \quad R = diag( p_R^1, \dots, p_R^l), p_R^i \geq 0, i = 1:l$$

$$(8) \quad Q = diag( p_Q^1, \dots, p_Q^n), p_Q^i \geq 0, i = 1:n$$

où :

-   l'indice i représente l'indice du coefficient dans le jeu de coefficients $p_R$ ou $p_Q$,
-   $l$ et $n$ sont respectivement les dimensions des matrices $R$ et $Q$.

De plus, les matrices $R$ et $Q$ sont des matrices symétriques définies positives c'est-à-dire dont les valeurs propres sont positives. Pour garantir cette condition, les matrices $R$ et $Q$ peuvent s'écrire comme notées ci-dessous :
$R=X^TX$ et $Q=Y^TY$, où les matrices X et Y sont des matrices diagonales. Notons que lorsque les matrices $R$ et $Q$ sont des matrices quelconques, les matrices X et Y sont des matrices triangulaires supérieures.
A l'issu de l'étape 42, avec les hypothèses faites, le vecteur p de coefficients à déterminer est défini par la relation suivante :

$$(9) \quad p=\left[\,p_R^T,p_Q^T\right]^T$$

Lors d'une étape 44, le critère à optimiser pour déterminer le vecteur p est choisi. Ici, ce critère est donné par la relation suivante :

$$(10) \qquad \min_{p\in\varDelta}\left\{f(p)=\sum_{k=1}^N \|z_k-\ \phi(\hat{x}_k,p)\|_2^2\right\}$$

où :

-   $\|...\|_2$ représente l'opération norme $L_2$, correspondant à la norme euclidienne,
-   « min » représente l'opération consistant à trouver le vecteur p qui minimise la fonction $f(p)$ ,
-   $\Delta$ est un ensemble de contraintes que doit vérifier le vecteur p qui minimise la fonction $f(p)$ .

La fonction $\phi$ qui apparaît dans le critère (10) est la même que celle introduite ci-dessus. Cette fonction $\phi$ permet d'obtenir l'estimation $\hat{z}_k$ de la variable $z_k$ à partir de l'estimation $\hat{x}_k$ comme indiqué dans la relation suivante :

$$(11) \quad \hat{z}_k=\phi(\hat{x}_k,p)$$

On notera que la fonction $\phi$ dépend également du vecteur p puisque l'estimation $\hat{x}_k$ qui constitue l'argument de la fonction $\phi$ est elle-même fonction du vecteur p. Pour cette raison, dans la relation ci-dessus, l'estimation $\hat{x}_k$ et le vecteur p sont indiqués comme argument de la fonction $\phi$.
Lors d'une étape 46, l'ensemble $\Delta$ des contraintes à respecter par le vecteur p est défini. Cet ensemble $\Delta$ comprend au moins une contrainte qui impose que l'estimation $\hat{z}_k$ et la variable $z_k$ soient proches l'une de l'autre. Plus précisément, cette contrainte impose ici que la trajectoire de la variable $z_k$ soit comprise, au moins en moyenne, dans un couloir 48 d'incertitude (figure 3) situé de part et d'autre de la trajectoire de l'estimation $\hat{z}_k$.
Ce couloir 48 est délimité par deux limites $L_{min}$ et $L_{max}$ situées, respectivement, de chaque côté de la trajectoire de l'estimation $\hat{z}_k$. Le couloir d'incertitude représente le fait que l'estimation $\hat{z}_k$ est uniquement connue à une incertitude près. Cette incertitude provient du fait qu'il existe une incertitude sur l'estimation $\hat{x}_k$ utilisée pour construire l'estimation $\hat{z}_k$. Ainsi, pour calibrer correctement l'observateur 18, il faut que pour la majorité des instants k, la variable $z_k$ soit comprise dans le couloir 48.
Dans le cas particulier représenté sur la figure 3, les limites $L_{max}$ et $L_{min}$ qui délimitent le couloir 48 sont séparées de la trajectoire de $\hat{z}_k$, respectivement, par des distances a et b. Dans le cas de la figure 3, ces distances a et b sont constantes au cours du temps.
De préférence, les distances a et b sont fonctions de l'incertitude sur l'estimation $\hat{z}_k$. Par exemple, les distances a et b sont d'autant plus grande que l'écart-type $\sigma_{\hat{z}_k}$ est grand. $\sigma_{\hat{z}_k}$ est l'écart type de l'erreur sur l'estimation $\hat{z}_k$. A titre d'exemple, ici, l'ensemble $\Delta$ comprend une contrainte qui impose que la moyenne des écarts entre la variable $z_k$ et l'estimation $\hat{z}_k$ soit inférieure à un seuil prédéterminé $S_1$. Cette contrainte s'exprime à l'aide de la relation suivante :

$$(11) \qquad \sqrt{\sum_{k=1}^{N} \|z_k - \hat{z}_k\|_2} \leq S_1$$

Le seuil $S_1$ est par exemple défini par la relation suivante :

$$(12) \quad S_1 = \sqrt{N}\, \alpha \sigma_{\hat{z}_k}$$

où :

- $N$ est le nombre d'instants $k$, et
- $\alpha$ est une constante, par exemple, égale à trois.

Pour chaque vecteur p minimisant potentiellement le critère (10), il est possible de déterminer l'écart-type $\sigma_{\hat{z}_k}$. En effet, lorsqu'un vecteur p est connu, les matrices R et Q sont également connues. A partir des matrices $R$ et $Q$, il est possible de calculer la matrice $P_{k/k}$ de la covariance de l'erreur d'estimation de $\hat{x}_k$. A partir de cette matrice $P_{k/k}$ il est possible de déterminer l'incertitude entachant l'estimation $\hat{x}_k$. Connaissant cette incertitude, il est possible également de déterminer l'incertitude sur l'estimation $\hat{z}_k$ puisque la fonction $\phi$ reliant ces deux estimations est connue. L'incertitude sur l'estimation $\hat{z}_k$ est alors exprimée sous la forme de l'écart type $\sigma_{\hat{z}_k}$.

Lors de l'étape 46, d'autres contraintes de l'ensemble $\Delta$ peuvent être définies. Par exemple, à partir du sens physique de certains des coefficients des jeux $p_R$ et $p_Q$, il est possible d'imposer des limites à ces coefficients. Par exemple, si des informations sur le bruit de mesure du capteur 10 sont connues, il est possible de borner à partir des ces informations les valeurs que peuvent prendre certains coefficients du jeu $p_R$ et $p_Q$.

Une fois l'ensemble $\Delta$ défini, lors d'une étape 50, le capteur 32 mesure la variable $z_k$ à N instants différents. Le nombre N d'instants est typiquement supérieur au nombre de coefficients à déterminer contenus dans le vecteur p.

Une fois les variables $z_k$ mesurées, celles-ci sont acquises par le calculateur 34. On initialise alors, lors d'une étape 52, les différentes variables et coefficients nécessaires pour minimiser le critère (10). Par exemple, la matrice initiale $P_{0/0}$ d'estimation de la covariance sur l'erreur de l'estimation $\hat{x}_0$ est définie par la relation suivante :

$$(13) \quad P_{0/0} = \lambda I, \lambda \gg 1$$

où :

- $I$ est la matrice identité, et
- $\lambda$ est une constante.

La constante $\lambda$ est réglée en fonction de la confiance que l'on a dans l'initialisation de $\hat{x}_0$.

Le choix d'une constate $\lambda$ beaucoup plus grande que un signifie que l'incertitude sur l'estimation initiale $\hat{x}_0$ est très grande.

Ici, l'estimation initiale $\hat{x}_0$ et la valeur initiale des coefficients du vecteur p sont tirées de façon aléatoire.

Ensuite, on procède à une étape 54 de détermination du vecteur p qui minimise le critère (10). Cette étape consiste à faire varier les valeurs du vecteur p jusqu'à trouver un vecteur p qui minimise le critère (10) et qui satisfasse en même temps les contraintes de l'ensemble $\Delta$. Cette étape est réalisée à l'aide d'un outil d'optimisation sous contraintes. De tels outils d'optimisation sous contraintes sont connus. Par exemple, un tel outil est en particulier la fonction « Fmincon » disponible dans l'environnement de développement « MATLAB® » pour le calcul numérique entre autres.

Une fois que le vecteur p qui minimise le critère (10) a été déterminé, lors de l'étape 56, les valeurs des coefficients de ce vecteur p sont utilisées pour calibrer les coefficients des matrices $R$ et $Q$ de l'observateur 18. Par exemple, lors de cette étape, les valeurs de ces coefficients sont copiées et enregistrées dans la mémoire 20.

Ensuite, lors d'une étape 58, le système 6 de localisation utilise les coefficients déterminés par le système 30 pour déterminer la position et l'orientation de l'objet 4 à partir des mesures réalisées par le capteur 10.

De nombreux autres modes de réalisation sont possibles. Par exemple, pour un même système physique et pour un même observateur, il est possible de faire des choix différents sur le nombre de coefficients de cet observateur à déterminer pour le calibrer. Par exemple, le nombre de coefficients peut encore être plus réduit en imposant aux différents coefficients de la matrice diagonale *R* ou *Q* d'être tous proportionnels à un même coefficient. A l'inverse, le procédé précédemment décrit s'applique également au cas où les matrices *Q* et *R* ne sont pas des matrices diagonales mais uniquement des matrices symétriques définies positives. Cette hypothèse augmente au contraire le nombre de coefficients à déterminer par le système 30.

Dans le critère (10) la norme euclidienne peut être remplacée par une autre norme.

D'autres contraintes que celles décrites précédemment peuvent être utilisées. Par exemple, il est possible d'imposer que la variable $z_k$ soit systématiquement comprise à l'intérieur du couloir d'incertitude 48 à chaque instant *k*. Cette contrainte est plus forte que celle décrite en regard de la figure 3 qui impose uniquement que la variable $z_k$ soit en moyenne comprise dans le couloir d'incertitude 48.

Des ensembles différents de contraintes peuvent être utilisés à différents instants. Par exemple, si à certains instants appartenant à un ensemble L l'incertitude sur la mesure de la variable $z_k$ est très faible, on peut alors choisir une contrainte plus forte à ces instants. Par exemple, si l'instant *k* appartient à l'ensemble L, alors il est possible d'imposer que la variable $z_k$ soit systématiquement comprise dans un couloir d'incertitude plus restreint. Pour les instants n'appartenant pas à l'ensemble L, la contrainte utilisée sera moins forte. Par exemple elle imposera uniquement que, en moyenne, la variable $z_k$ soit comprise dans le couloir d'incertitude 48. Le couloir d'incertitude utilisé pour définir la contrainte lorsque l'instant k appartient à l'ensemble L n'est pas nécessairement le même que celui utilisé pour définir la contrainte lorsque l'instant k n'appartient pas à cet ensemble L.

D'autres contraintes supplémentaires que celles imposant que la variable $z_k$ soit dans le couloir d'incertitude de la trajectoire de l'estimation $\hat{z}_k$ peuvent être utilisées. Par exemple, il est possible de définir des contraintes qui imposent une faible variation de l'estimation $\hat{z}_k$ entre deux instants successifs.

D'autres choix sont possibles pour initialiser l'optimisation du critère (10) que ceux décrits ci-dessus. Par exemple, si la valeur initiale de la variable $x_0$ est connue, cette valeur est utilisée pour initialiser la variable $x_0$ et les coefficients de la matrice $P_0$ sont réglés en fonction de la confiance en cette valeur.

L'incertitude sur l'estimation $\hat{z}_k$ peut être obtenue par d'autres moyens qu'à partir des observations de l'observateur. Par exemple, cette incertitude peut être déterminée expérimentalement ou à partir d'autres systèmes d'équations que ceux définissant l'observateur 18.

La variable mesurée $z_k$ et la mesure $y_k$ peuvent être confondues. Ainsi, il n'est pas nécessaire de recourir à un capteur supplémentaire tel que le capteur 32.

Le procédé de calibration décrit ci-dessus s'applique également au filtre de Kalman étendu (connu sous le terme anglais de « Extended Kalman Filter ») ainsi qu'au filtre de Kalman non parfumé plus connus sous l'acronyme UKF (Unscented Kalman Filter) ou à tout autre filtre récursif présentant des paramètres à régler. De manière plus générale, le procédé de calibration décrit ci-dessus peut être utilisé pour calibrer tout observateur d'une variable d'état dont des coefficients doivent être déterminés au préalable. Ainsi, les coefficients susceptibles d'être déterminés à l'aide du procédé ci-dessus ne sont pas limités à ceux d'une matrice de covariance.

L'invention a été décrite dans le cadre d'une application donnée. Cependant, elle n'est pas limitée à ce type d'application. D'autres applications peuvent être prises en compte, comme, par exemple, obtenir des mesures dans le sondage des fonds des mers, pour lequel le système physique permet d'avoir des mesures représentatives des distances surfaces/fonds des mers ou aux profondeurs des mers.

**Revendications**

1. Procédé de calibration des coefficients d'un observateur d'une variable d'état $x_k$ d'un système physique à partir de mesures de grandeurs physiques de ce système, où l'indice k identifie l'instant de mesure,
   **caractérisé en ce que** le procédé comporte :

   - la mesure (50) d'une variable $z_k$ du système physique à N instants différents, cette variable $z_k$ étant fonction de la variable d'état $x_k$,
   - la détermination (54) d'un vecteur p de coefficients qui minimise le critère suivant en respectant un ensemble prédéterminé $\Delta$ de contraintes :

$$\left\{ f(p) = \sum_{k=1}^{N} \| z_k - \phi(\hat{x}_k, p) \|^2 \right\}$$

où :

• $\hat{x}_k$ est l'estimation de la variable $x_k$ construite par l'observateur calibré avec les coefficients du vecteur p à l'instant k,

• $\phi$ est une fonction connue qui relie l'estimation $\hat{x}_k$ à une estimation $\hat{z}_k$ de la variable $z_k$,

• $\|...\|^2$ est une norme de la différence entre $z_k$ et $\phi(\hat{x}_k,p)$, et

• la ou les contraintes de l'ensemble $\Delta$ imposent que la trajectoire de la variable $z_k$ soit comprise dans un couloir d'incertitude situé de part et d'autre de la trajectoire de l'estimation $\hat{z}_k$ au moins pour la majorité des instants *k*.

2. Procédé selon la revendication 1, dans lequel l'ensemble $\Delta$ comprend une contrainte qui impose que la variable $z_k$ soit comprise entre $\hat{z}_k$-*a* et $\hat{z}_k$+*b*, où a et b sont des constantes positives prédéterminées.

3. Procédé selon la revendication 2, dans lequel les constantes a et b sont proportionnelles à un écart-type $\sigma_{\hat{z}_k}$ représentatif de l'incertitude sur l'estimation $\hat{z}_k$.

4. Procédé selon l'une quelconque des revendications précédentes, dans lequel l'ensemble $\Delta$ de contraintes comprend une contrainte qui impose que la moyenne des écarts entre la variable $z_k$ et son estimation $\hat{z}_k$ soit inférieure à un seuil prédéterminé $S_1$.

5. Procédé selon la revendication 4, dans lequel le seuil $S_1$ est fonction de l'incertitude sur l'estimation de $\hat{z}_k$.

6. Procédé selon l'une quelconque des revendications précédentes, dans lequel l'observateur est un filtre de Kalman, un filtre de Kalman étendu ou un filtre de Kalman non parfumé et les coefficients à calibrer sont les coefficients des matrices de covariances *R* et *Q* de ce filtre.

7. Procédé selon la revendication 6, dans lequel les matrices *R* et Q sont choisies diagonales.

8. Support (36) d'enregistrement d'informations, **caractérisé en ce qu'**il comporte des instructions pour l'exécution d'un procédé conforme à l'une quelconque des revendications précédentes, lorsque ces instructions sont exécutées par un calculateur électronique.

9. Système de calibration des coefficients d'un observateur d'une variable d'état $x_k$ d'un système physique à partir de mesures de grandeurs physiques de ce système, où l'indice k identifie l'instant de mesure, **caractérisé en ce que** ce système comporte :

- au moins un capteur (32) apte à mesurer une variable $z_k$ du système physique à *N* instants différents, cette variable $z_k$ étant fonction de la variable d'état $x_k$ , et

- un calculateur (34) apte à déterminer un vecteur p de coefficients qui minimise le critère suivant en respectant un ensemble prédéterminé $\Delta$ de contraintes :

$$\left\{ f(p)=\sum_{k=1}^{N} \| z_k - \phi(\hat{x}_k,p)\|^2 \right\}$$

où :

• $\hat{x}_k$ est l'estimation de la variable $x_k$ construite par l'observateur calibré avec les coefficients du vecteur p à l'instant k,

• $\phi$ est une fonction connue qui relie l'estimation $\hat{x}_k$ à une estimation $\hat{z}_k$ de la variable $z_k$ ,

• $\|...\|^2$ est une norme de la différence entre $z_k$ et $\phi(\hat{x}_k,p)$, et

• la ou les contraintes de l'ensemble $\Delta$ impose que la trajectoire de la variable $z_k$ soit comprise dans un couloir d'incertitude situé de part et d'autre de la trajectoire de l'estimation $\hat{z}_k$ au moins pour la majorité des *instants k.*

Fig. 1

Fig. 2

Fig. 3

# EP 2 372 912 A1

Europäisches
Patentamt

European
Patent Office

Office européen
des brevets

**RAPPORT DE RECHERCHE EUROPEENNE**

Numéro de la demande

EP 11 16 0085

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | BERNT M. AKESSON ET AL.: "A Tool for Kalman Filter Tuning", COMPUTER AIDED CHEMICAL ENGINEERING, [Online] 21 février 2008 (2008-02-21), pages 859-864, XP002616486, Amsterdam, The Netherlands DOI: 10.1016/S1570-7946(07)80166-0 [extrait le 2011-01-10] * le document en entier * ----- | 1,9 | INV. H03H21/00 A61B5/06 |
| A | YUAN GAO ET AL: "Self-Tuning Multisensor Weighted Measurement Fusion Kalman Filter", IEEE TRANSACTIONS ON AEROSPACE AND ELECTRONIC SYSTEMS, IEEE SERVICE CENTER, PISCATAWAY, NJ, US, vol. 45, no. 1, 1 janvier 2009 (2009-01-01), pages 179-191, XP011268019, ISSN: 0018-9251, DOI: 10.1109/TAES.2009.4805272 * alinéas [000I], [0III] - [00IV] * ----- | 1,9 | |
| A | XIAOHE LI ET AL: "Object tracking using an adaptive Kalman filter combined with mean shift", OPTICAL ENGINEERING SPIE - THE INTERNATIONAL SOCIETY FOR OPTICAL ENGINEERING USA, vol. 49, no. 2, février 2010 (2010-02), pages 020503-1-020503-3, XP002616487, ISSN: 0091-3286, DOI: 10.1117/1.3327281 * alinéa [0003] * ----- | 1,9 | DOMAINES TECHNIQUES RECHERCHES (IPC)<br><br>H03H<br>A61B |
| A | US 2003/115232 A1 (LIPP JOHN [US] LIPP JOHN I [US]) 19 juin 2003 (2003-06-19) * alinéas [0036] - [0095] * ----- | 1,9 | |
| | -/-- | | |

Le présent rapport a été établi pour toutes les revendications

| Lieu de la recherche | Date d'achèvement de la recherche | Examinateur |
|---|---|---|
| La Haye | 29 août 2011 | De La Pinta, Luis |

EPO FORM 1503 03.82 (P04C02)

Europäisches
Patentamt

European
Patent Office

Office européen
des brevets

**RAPPORT DE RECHERCHE EUROPEENNE**

Numéro de la demande

EP 11 16 0085

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A,D | CA 2 266 282 A1 (NNEME NNEME LEANDRE [CA]; O'SHEA JULES [CA]) 19 septembre 2000 (2000-09-19) * abrégé * ----- | 1,9 | |

DOMAINES TECHNIQUES RECHERCHES (IPC)

Le présent rapport a été établi pour toutes les revendications

| Lieu de la recherche | Date d'achèvement de la recherche | Examinateur |
|---|---|---|
| La Haye | 29 août 2011 | De La Pinta, Luis |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons
...........................................................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 11 16 0085

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

29-08-2011

| Document brevet cité au rapport de recherche | Date de publication | Membre(s) de la famille de brevet(s) | Date de publication |
|---|---|---|---|
| US 2003115232 A1 | 19-06-2003 | AUCUN | |
| CA 2266282 A1 | 19-09-2000 | AUCUN | |

EPO FORM P0460

Pour tout renseignement concernant cette annexe : voir Journal Officiel de l'Office européen des brevets, No.12/82

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- CA 2226282

- EP 1502544 A